(19) Europäisches Patentamt
European Patent Office
Office européen des brevets

(11) **EP 3 074 368 B1**

(12) **FASCICULE DE BREVET EUROPEEN**

(45) Date de publication et mention de la délivrance du brevet:
**27.12.2017 Bulletin 2017/52**

(21) Numéro de dépôt: **14781245.7**

(22) Date de dépôt: **08.10.2014**

(51) Int Cl.:
***C07C 1/24*** *(2006.01)*

(86) Numéro de dépôt international:
**PCT/EP2014/071551**

(87) Numéro de publication internationale:
**WO 2015/078625 (04.06.2015 Gazette 2015/22)**

(54) **PROCEDE DE DESHYDRATATION D'UN MELANGE CONTENANT DE L'ETHANOL ET DU N-PROPANOL**

VERFAHREN ZUR ENTWÄSSERUNG EINER MISCHUNG MIT ETHANOL UND N-PROPANOL

METHOD FOR DEHYDRATING A MIXTURE CONTAINING ETHANOL AND N-PROPANOL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2013 FR 1361685**

(43) Date de publication de la demande:
**05.10.2016 Bulletin 2016/40**

(73) Titulaires:
• **IFP Energies nouvelles**
  **92500 Rueil-Malmaison (FR)**
• **Total Research & Technology Feluy**
  **7181 Seneffe (BE)**

(72) Inventeurs:
• **ARIBERT, Nicolas**
  **F-38430 Moirans (FR)**
• **BRANDHORST, Laure**
  **F-69008 Lyon (FR)**
• **COUPARD, Vincent**
  **F-69100 Villeurbanne (FR)**
• **MAURY, Sylvie**
  **F-69440 Saint Maurice d'argoire (FR)**
• **VIVIEN, Tom**
  **F-69007 Lyon (FR)**

(74) Mandataire: **Schmitt, Nicolas A.J. et al**
  **IFP Energies nouvelles**
  **Département Propriété Industrielle**
  **Rond-point de l'Echangeur de Solaize**
  **BP3**
  **69360 Solaize (FR)**

(56) Documents cités:
**EP-A1- 0 498 573     WO-A1-2011/162717**

## Description

La présente invention concerne un procédé de production d'un mélange d'éthylène et de propylène à partir d'un mélange aqueux contenant de l'éthanol et du n-propanol. Le procédé selon l'invention permet en particulier de traiter un mélange aqueux d'éthanol et de n-propanol qui est obtenu à partir de ressources renouvelables comme par exemple de la biomasse.

## Etat de la technique

Les oléfines légères sont des intermédiaires importants dans l'industrie chimique et sont principalement produites par craquage catalytique ou vapocraquage d'hydrocarbures.

Par exemple le propylène est largement utilisé dans l'industrie chimique dans la production de l'acrylonitrile, l'acide acrylique et surtout du polypropylène. Il en est de même pour l'éthylène qui est utilisé pour la synthèse d'un grand nombre de polymères et de matières plastiques, tels que le polychlorure de vinyle (PVC), le polyéthylène (PE) et le polyester via le monoéthylène glycol(MEG).

Pour répondre aux enjeux futurs liés à la diminution des ressources pétrolières et aux préoccupations environnementales, de nombreuses recherches sont actuellement menées pour développer des technologies alternatives de synthèses de ces intermédiaires à partir de ressources renouvelables.

Une des voies les plus étudiées actuellement pour la production d'oléfines est celle de la déshydratation catalytique des alcools.

La réaction de déshydratation de l'éthanol en éthylène est connue et détaillée depuis la fin du XIXème siècle. Il est connu que cette réaction est très endothermique, équilibrée et déplacée vers l'éthylène à haute température. Le catalyseur de référence souvent utilisé est un catalyseur monofonctionnel à caractère acide. L'alumine gamma est le catalyseur le plus cité. L'article "The Deshydration of Alcohols over Alumina. I: The reaction scheme", H. Knözinger, R. Köhne, Journal of Catalysis (1966), 5, 264-270 est considéré comme la publication de base sur les travaux de déshydratation des alcools dont l'éthanol.

Les zéolithes sont également utilisées pour cette application, et en particulier la ZSM5 depuis les années 1980, comme décrit dans "Reactions of ethanol over ZSM-5",S.N. Chaudhuri & al., Journal of Molecular Catalysis 62:289-295 (1990).

La voie de déshydratation des alcools répond bien à la problématique de produire de manière alternative et de façon "verte" des oléfines. En effet l'éthanol et ses homologues supérieurs peuvent être synthétisés par fermentation de sucres obtenus à partir de sources renouvelables telles que des plantes amylacées ou sucrières (e.g. le maïs, la canne à sucre respectivement) ou à partir de biomasse lignocellulosique telle que par exemple des copeaux de bois ou des résidus de cultures (paille de blé).

On connaît dans l'état de la technique le document WO 2004/078336 qui divulgue un procédé de production d'a-oléfines par déshydratation d'alcools linéaires ou ramifiés ayant 4 à 14 atomes de carbones en présence d'une alumine $\gamma$ comprenant:

- un volume poreux supérieur à 0,9 mL/g (déterminé selon la méthode DIN 66133);
- des pores dans le domaine mésoporeux (diamètre maximum compris entre 20 et 90 Å);
- des pores dans le domaine macroporeux (diamètre maximum supérieur à 250 Å).

La demande WO 2011/162717 a pour objet un procédé de production d'oléfines par déshydratation d'un mélange d'alcools au moyen d'un catalyseur comprenant une zéolite dopée avec un élément métallique choisi parmi : Mg, Ca, Ba, Sr, Cr, Mn, Fe, Co, Ni, Cu, Zn, Ga, Ce, Ag, Bi, Ti, V, Zr, Mo, W, Li, La. Il est envisagé dans ce document d'utiliser des alcools obtenus par le traitement de la biomasse.

On cite enfin la demande de brevet FR 2 961 202 qui décrit un procédé de production d'oléfines en C4 à partir d'une charge de monoalcool en C4 par réaction de déshydratation dudit alcool en présence d'un catalyseur à base d'alumine à porosité contrôlée.

Un but de la présente invention est de fournir un procédé de production d'un mélange d'éthylène et de propylène directement à partir d'un mélange contenant de l'eau, de l'éthanol et du n-propanol. Selon l'invention, le procédé s'applique notamment à un mélange d'éthanol et de n-propanol d'origine biologique obtenu à partir du traitement de la biomasse.

## Résumé de l'invention

La présente invention concerne donc un procédé de production d'un mélange d'éthylène et de propylène à partir d'un mélange contenant de l'éthanol, du n-propanol et ayant une teneur en eau comprise entre 30 et 75% poids par rapport au poids total du mélange, dans lequel:

a) on met en contact dans une unité de déshydratation le mélange avec un catalyseur de déshydratation choisi parmi:

- une alumine (A) ayant une surface spécifique BET mesurée selon la norme ASTM D 3663-03 comprise entre 200 et 350 m$^2$/g, un diamètre moyen mésoporeux compris entre 5 et 15 nm, une teneur en sodium inférieure à 50 ppm poids et une teneur en soufre inférieure à 40 ppm poids; et
- une alumine (B) ayant une surface spécifique BET mesurée selon la norme ASTM D 3663-03 comprise entre 130 et 180 m$^2$/g, un diamètre moyen mésoporeux compris entre 14 et 20 nm, une teneur en sodium comprise entre 300 et 600 ppm poids et une teneur en soufre comprise entre 800 et 1300 ppm poids;

la mise en contact étant effectuée à une température comprise entre 350 et 500°C, à une pression totale comprise entre 0,2 et 2 MPa et avec une vitesse pondérale horaire (pph) définie comme étant le rapport du débit massique en éthanol et n-propanol sur la masse de catalyseur comprise entre 1 et 10 h$^{-1}$

b) on soutire un effluent contenant de l'éthylène et du propylène de ladite unité de déshydratation.

Le procédé selon l'invention permet ainsi de répondre aux enjeux de la transition énergétique en proposant un procédé alternatif à la filière pétrochimique pour la production d'éthylène et de propylène, à partir d'un mélange d'alcools qui peut être d'origine biologique.

De manière surprenante, les inventeurs ont trouvé qu'un catalyseur ayant les caractéristiques mentionnées ci-dessus présente une forte activité en déshydratation, tout en étant également sélectif vers les oléfines recherchées.

Le procédé selon l'invention présente l'avantage qu'il s'affranchit d'une étape de séparation de l'éthanol et du n-propanol avant l'étape de déshydratation. En effet, grâce aux catalyseurs selon l'invention, il est possible, en une seule étape catalytique, de déshydrater le mélange desdits alcools et ainsi de produire un effluent comprenant de l'éthylène et du propylène.

Le procédé est donc avantageux du point de vue économique et énergétique lorsqu'il est envisagé de produire de l'éthylène et du propylène pour la pétrochimie. En effet il n'est pas nécessaire grâce au procédé de réaliser une séparation préalable de l'éthanol et du n-propanol du mélange aqueux avant la déshydratation. Cette séparation des deux alcools est particulièrement coûteuse en investissement car pour être efficace, du fait de l'existence de compositions azéotropiques, elle requiert non seulement de concentrer le mélange d'alcools de manière à obtenir une concentration en alcools supérieure à 50% poids, mais également de mettre en oeuvre une colonne de distillation ayant un grand nombre de plateaux. Ainsi un avantage du procédé selon l'invention est qu'il permet de produire de l'éthylène et du propylène pour la pétrochimie en mettant en jeu un nombre plus faible d'opérations unitaires d'où un avantage économique non négligeable. Ainsi après l'étape de déshydratation selon l'invention, on obtient un flux contenant du propylène et de l'éthylène qui sont par contre facilement séparables par distillation.

Le procédé selon l'invention permet de déshydrater un mélange contenant de l'eau, de l'éthanol et du n-propanol et d'utiliser avantageusement l'eau contenue dans ledit mélange comme fluide thermique lors de l'étape de déshydratation. La réaction de déshydratation est fortement endothermique et le fait que la charge soit diluée à l'eau permet de réduire l'endotherme de réaction et ainsi d'effectuer la réaction avec un nombre limité de réacteurs. L'eau permet également grâce aux échangeurs charge/effluent de récupérer l'énergie de condensation des produits de réaction pour amener à la température requise la charge à déshydrater.

De préférence, l'alumine (A) a une surface spécifique BET mesurée selon la norme ASTM D 3663-03 comprise entre 200 et 280 m$^2$/g et de manière plus préférée comprise entre 200 et 230 m$^2$/g.

De préférence l'alumine (A) a un diamètre moyen mésoporeux compris entre 6 et 12 nm et de manière plus préférée comprise entre 7 et 11 nm.

De préférence, l'alumine (B) a une surface spécifique BET mesurée selon la norme ASTM D 3663-03 comprise entre 150 et 180 m$^2$/g.

De préférence l'alumine (B) a un diamètre moyen mésoporeux compris entre 15 et 20 nm.

Selon un mode de réalisation très préféré, les catalyseurs (A) et (B) selon l'invention sont des alumines gamma. De manière plus préférée, les catalyseurs selon l'invention sont constitués d'alumine gamma.

Les alumines selon l'invention peuvent être préparées par toute méthode connue de l'Homme du métier. Par exemple elles peuvent être obtenues à partir d'une alumine gel (ou gel d'alumine) qui comprend essentiellement un précurseur du type oxy(hydroxyde) d'aluminium (AlO(OH)) - également dénommé boehmite. Le gel d'alumine (ou autrement dénommé gel de boehmite) est par exemple synthétisé par précipitation de solutions basiques et/ou acides de sels d'aluminium induite par changement de pH ou tout autre méthode connue de l'homme de métier (P. Euzen, P. Raybaud, X. Krokidis, H. Toulhoat, J.L. Le Loarer, J.P. Jolivet, C. Froidefond, Alumina, in Handbook of Porous Solids, Eds F. Schüth, K.S.W. Sing, J. Weitkamp, Wiley-VCH, Weinheim, Germany, 2002, pp. 1591-1677). Le gel obtenu est ensuite soumis à une ou plusieurs étapes de traitement thermique qui comprend notamment un séchage et une calcination afin de former une alumine ayant les caractéristiques structurales requise selon l'invention. Il est à noter que les alumines selon l'invention peuvent également être obtenues à partir d'autres précurseurs d'alumine tels que par exemple la bauxite, la bayerite ou par oxydation de l'aluminium.

Selon un mode de réalisation particulier, avant l'étape a), on procède à une étape de réduction de la teneur en eau (étape de séchage) du mélange éthanol-n-propanol-eau qui comprend une mise en contact, dans une colonne d'extraction liquide-liquide, du mélange avec une coupe aromatique comprenant un mélange de composés aromatiques ayant 7 à 10 atomes de carbone. La coupe aromatique est de préférence un mélange de 1,3,5-triméthylbenzène et de 1,2,4-triméthylbenzène. Alternativement, la coupe aromatique est une coupe reformat lourd issue d'une séparation du produit de reformage d'une coupe essence. La mise en contact du mélange d'alcools avec la coupe aromatique dans la colonne d'extraction est faite de préférence à contre-courant et de préférence avec une température de mise en contact comprise entre 70 et 90°C.

Cette étape d'extraction permet de séparer de la colonne une fraction aqueuse et une fraction organique contenant la coupe aromatique, de l'éthanol, du n-propanol. La fraction organique est ensuite envoyée dans une colonne de distillation configurée pour séparer un effluent contenant la coupe aromatique et un effluent contenant de l'éthanol, du n-propanol. Enfin on envoie l'effluent contenant de l'éthanol et du n-propanol dans l'unité de déshydratation de l'étape a) avec un appoint d'eau.

De manière alternative, avant l'étape a), on procède à une étape de réduction de la teneur en eau du mélange éthanol/n-propanol/eau, qui consiste à envoyer le mélange dans une colonne de distillation de manière à séparer une fraction aqueuse et un effluent ayant une teneur réduite en eau et contenant de l'éthanol et du n-propanol. L'effluent ainsi récupéré est traité dans l'unité de déshydratation conformément à l'étape a).

De préférence le mélange contenant de l'éthanol, du n-propanol est issu d'une estérification de l'acide propanoïque avec de l'éthanol puis hydrogénation de l'ester (propanoate d'éthyle). Il existe également des voies métaboliques permettant le passage de l'acide propanoïque à l'alcool par catalyse enzymatique. En particulier, l'acide propanoïque est le produit d'une fermentation de type propionique d'un substrat choisi parmi des sucres, le glycérol, l'acide lactique, l'acide malique. De préférence l'acide propanoïque provient de la fermentation de sucres obtenus par le traitement de la biomasse, par exemple lignocellulosique. Une autre voie possible est la fermentation du $CO$, $H_2$, $CO_2$ par voie bactérienne anaérobique, par exemple via *Clostridium ljungdahlii* qui produit des mélanges d'alcools contenant du n-propanol. L'éthanol est par exemple le produit de la fermentation de sucres obtenus à partir de sources renouvelables telles que des plantes amylacées ou sucrières (e.g. le maïs, la canne à sucre respectivement) ou à partir de biomasse lignocellulosique telle que par exemple des copeaux de bois ou des résidus de cultures (paille de blé).

De préférence, le procédé de déshydratation est effectué dans deux réacteurs adiabatiques en série.

## Description détaillée de l'invention

La charge traitée dans le procédé selon l'invention est un mélange contenant entre 30 et 75% poids d'eau par rapport au poids total du mélange, de l'éthanol et de n-propanol.

Le mélange qui est traité par déshydratation selon l'invention peut comprendre n'importe quelle proportion d'éthanol et de n-propanol. De préférence, le mélange contient entre 1 et 75% poids d'éthanol et entre 99 et 25% poids de n-propanol par rapport au poids total éthanol et n-propanol.

La charge d'éthanol/n-propanol provient de préférence de ressources renouvelables. De préférence la charge est un produit issu du traitement biologique de la biomasse. De manière préférée, la charge d'éthanol/n-propanol est une charge produite par estérification de l'acide propanoïque avec de l'éthanol puis hydrogénation de l'ester. L'acide propanoïque est de préférence d'origine biologique. L'acide propanoïque (également appelé acide propionique) est obtenue par une fermentation dite « propionique » de substrats variés, comme le glycérol, l'acide lactique, l'acide malique et les sucres. De manière préférée, l'acide propanoïque est issu de la fermentation de sucres qui sont produits à partir des cultures de plantes sucrières comme la canne à sucre, des betteraves, ou encore des plantes amylacées ou du traitement de la biomasse lignocellulosique ou de cellulose hydrolysée. On connaît des microorganismes du genre *Propionibacterium* qui sont capables de transformer les substrats mentionnés ci-dessus en acide propanoïque.

Selon l'invention la conversion du mélange d'éthanol et de n-propanol respectivement en éthylène et propylène est réalisée, par exemple dans un réacteur, par mise en contact de la charge avec un catalyseur choisi parmi :

- une alumine (A) ayant une surface spécifique BET mesurée selon la norme ASTM D 3663-03 comprise entre 200 et 350 m$^2$/g, un diamètre moyen mésoporeux compris entre 5 et 15 nm, une teneur en sodium inférieure à 50 ppm poids et une teneur en soufre inférieure à 40 ppm poids; et
- une alumine (B) ayant une surface spécifique BET mesurée selon la norme ASTM D 3663-03 comprise entre 130 et 180 m$^2$/g, un diamètre moyen mésoporeux compris entre 14 et 20 nm, une teneur en sodium comprise entre 300 et 600 ppm poids et une teneur en soufre comprise entre 800 et 1300 ppm poids;

La détermination du diamètre moyen mésoporeux est faite par porosimétrie au mercure selon la norme ASTM D 4284-03 avec un angle de contact de 140°. L'analyse de porosimétrie au mercure correspond à l'intrusion d'un volume de mercure caractéristique de l'existence de mésopores et de macropores dans ledit catalyseur selon la norme ASTM D4284-03, les pores étant supposés de forme cylindrique. Cette technique permet d'accéder à la valeur du volume mercure mésoporeux défini comme étant le volume de mercure adsorbé par l'ensemble des pores ayant un diamètre compris dans la gamme des mésopores, à savoir compris entre 2 et 50 nm. Ce diamètre moyen mésoporeux est obtenu à partir de la courbe dérivée dV/dlog(D) (V étant le volume de mercure adsorbé et D le diamètre des pores) en fonction du diamètre des pores D et correspond à l'ordonnée pour laquelle l'abscisse dV/dlog(D) est maximal.

Le catalyseur de déshydratation utilisé dans l'étape a) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés.

Ledit catalyseur de déshydratation utilisé dans l'étape a) du procédé selon l'invention est avantageusement mis en oeuvre dans au moins un réacteur, en lit fixe ou en lit mobile.

Dans le cadre de l'invention, le catalyseur peut inclure au moins une matrice de type oxyde, également appelé liant. Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon.

Selon un mode de réalisation préféré, l'alumine dénommée "A" a une surface spécifique BET mesurée selon la norme ASTM D 3663-03 comprise entre 200 et 280 m$^2$/g et de manière préférée entre 200 et 230 m$^2$/g. De préférence l'alumine dénommée "A" a un diamètre moyen mésoporeux compris entre 6 et 12 nm et de manière plus préférée comprise entre 7 et 11 nm.

Selon un autre mode de réalisation, l'alumine dénommée "B" a une surface spécifique BET mesurée selon la norme ASTM D 3663-03 comprise entre 150 et 180 m$^2$/g. De préférence l'alumine dénommée "B" a un diamètre moyen mésoporeux compris entre 15 et 20 nm.

De préférence, les catalyseurs selon l'invention, avant leur mise en oeuvre sont soumis à une étape de calcination qui a pour objectif d'éliminer les espèces éventuellement adsorbées à leur surface.

L'étape de calcination consiste par exemple à porter le catalyseur à une température d'au moins 500°C sous un flux d'air ou d'azote pendant au moins 1 heure.

La réaction de déshydratation mettant en jeu les réactions suivantes :

$$C_2H_6O \rightarrow CH_2=CH_2 + H_2O$$

et

$$C_3H_8O \rightarrow CH_3\text{-}CH=CH_2 + H_2O$$

est réalisée :

- à une température comprise entre 350 et 500°C, de préférence comprise entre 350 et 450°C et de manière plus préférée comprise entre 375 et 425°C ;
- à une pression totale comprise entre 0,2 et 2 MPa, de préférence comprise entre 0,2 et 1 MPa et de manière plus préférée comprise entre 0,2 et 0,7 MPa ;
- et avec une vitesse pondérale horaire (pph), qui est définie comme étant le rapport du débit massique en alcools (i.e. éthanol et n-propanol) sur la masse de catalyseur, comprise entre 1 et 10 h$^{-1}$, de préférence comprise entre 2 et 8 h$^{-1}$.

Les conversions de l'éthanol et du n-propanol sont avantageusement supérieures à 90%, de préférence supérieures à 95% et de manière plus préférée supérieures à 99%.

Les conversions sont calculées au moyen de la formule suivante:

$$\text{Conversion alcool} = \left(1 - \frac{mC_{\text{alcool sortie}}}{mC_{\text{alcool entrée}}}\right) * 100$$

$$\text{mC}_{alcool} = \text{Masse alcool} * \frac{\text{Masse molaire Carbone}}{\text{Masse molaire alcool}}$$

et avec

Il a été trouvé que les catalyseurs mis en oeuvre selon l'invention permettent également d'atteindre des sélectivités vers les oléfines recherchées (éthylène et propylène) supérieure à 95%, de préférence supérieures à 99%. Les sélectivités en éthylène et propylène sont calculées en équivalent carbone massique et par rapport à l'alcool correspondant selon les formules :

$$\text{Sélectivité}_{éthylène} = \left( \frac{\text{mC}_{éthylène\ sortie}}{\text{mC}_{éthanol\ entrée} - \text{mC}_{éthanol\ sortie}} \right) * 100$$

et

$$\text{Sélectivité}_{propylène} = \left( \frac{\text{mC}_{propylène\ sortie}}{\text{mC}_{n\text{-}propanol\ entrée} - \text{mC}_{n\text{-}propanol\ sortie}} \right) * 100$$

La réaction de déshydratation est généralement conduite dans une unité comprenant au moins un réacteur isotherme ou adiabatique contenant un lit, par exemple fixe, de catalyseur de déshydratation.

De préférence, la réaction de déshydratation est mise en oeuvre dans deux réacteurs adiabatiques en série, comme décrit dans le document FR 2 978 146.

Selon ce mode de réalisation préféré, la charge comprenant le mélange d'éthanol et d'n-propanol est mélangée avec une partie du flux d'eau purifiée recyclée et une partie du flux d'éthanol et n-propanol qui n'ont pas été convertis, issu d'une zone de purification. Le mélange est introduit sous pression dans un premier échangeur gaz/liquide dans lequel ledit mélange subit un échange de chaleur avec l'effluent issu du dernier réacteur adiabatique. La chaleur latente ou enthalpie de condensation de l'effluent issu du dernier réacteur adiabatique est utilisée pour vaporiser la charge d'alcools en mélange avec le flux d'eau purifié recyclé et un flux d'éthanol/n-propanol non convertis (non déshydratés), sans apport de chaleur externe.

La charge d'alcools en mélange avec un flux d'eau purifiée recyclée et un flux d'éthanol/n-propanol non convertis (non déshydratés) est ensuite généralement envoyée dans un compresseur.

Ledit mélange vaporisé et comprimé est ensuite envoyé dans un second échangeur de type monophasique gaz, dans lequel ledit mélange est chauffé grâce à un échange de chaleur avec l'effluent issu du dernier réacteur adiabatique. Dans ledit échangeur, par exemple de type monophasique gaz, ladite charge vaporisée et comprimée est surchauffée et l'effluent à l'état gazeux, issu du dernier réacteur adiabatique est "désurchauffé" sans être condensé.

Ledit mélange de la charge et des deux flux, vaporisé, comprimé et chauffé dans l'échangeur de type monophasique gaz est ensuite chauffé, par exemple dans un four de manière à l'amener à une température d'entrée dans le premier réacteur adiabatique de déshydratation compatible avec la température de la réaction de déshydratation. L'effluent issu du premier réacteur est envoyé dans un deuxième moyen de chauffe avant d'être introduit dans le deuxième réacteur adiabatique de déshydratation.

L'effluent issu du deuxième réacteur subit ensuite les deux échanges successifs décrits précédemment dans les échangeurs.

L'effluent après passage dans le premier échangeur est envoyé dans une colonne de séparation gaz/liquide où il est séparé en un effluent comprenant de l'éthylène et du propylène et un effluent comprenant de l'eau. Une partie de l'effluent comprenant de l'eau est éventuellement recyclée, après refroidissement, dans la colonne de séparation.

La partie de l'effluent comprenant de l'eau non recyclée dans la colonne est envoyée dans une étape de purification et de séparation. Au moins un flux d'eau purifiée et au moins un flux d'éthanol/n-propanol non convertis sont ensuite séparés. Une partie dudit flux d'éthanol/n-propanol non réagis issu de l'étape de purification est mélangée avec au moins une partie du flux d'eau purifiée recyclée. Le mélange de ces deux flux est effectué en amont du premier échangeur, avec la charge aqueuse d'alcools à déshydrater.

Le procédé selon l'invention permet avantageusement de traiter un mélange contenant de l'éthanol et du n-propanol qui est par hydrogénation du propanoate d'éthyle lui-même obtenu par estérification de l'acide propanoïque avec de l'éthanol. De préférence, l'acide propanoïque provient d'une fermentation de type propionique d'un substrat choisi parmi des sucres, le glycérol, l'acide lactique, l'acide malique au moyen de microorganismes du genre *Propionibacterium.* De préférence, l'acide propanoïque est produit par fermentation de sucres fermentiscibles obtenus après prétraitement d'une biomasse du type lignocellulosique et hydrolyse enzymatique. La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries). De préférence l'éthanol est le produit de la fermentation de sucres obtenus à partir de sources renouvelables telles que des plantes amylacées ou sucrières (e.g. le maïs, la canne à sucre respectivement) ou à partir de biomasse lignocellulosique telle que par exemple des copeaux de bois ou des résidus de cultures (paille de blé).

Dans le cas de figure où la charge traitée est une solution aqueuse contenant de l'éthanol, du n-propanol avec plus de 80% poids en eau par rapport au poids total de la solution aqueuse, le procédé selon l'invention comprend avant l'étape a) de déshydratation de l'éthanol et du n-propanol, une étape de séparation de l'eau (ou séchage) de la solution aqueuse. Cette étape peut être réalisée selon toute technique connue de l'homme du métier.

Par exemple selon un premier mode de réalisation, cette séparation consiste en une distillation dans une colonne. Selon un second mode de réalisation, l'étape de séparation consiste en une étape d'extraction par solvant au moyen d'une coupe aromatique comprenant un mélange de composés aromatiques ayant 7 à 10 atomes de carbone. De préférence, la coupe aromatique comprend un mélange de 1,3,5-triméthylbenzène et de 1,2,4-triméthylbenzène. Cette extraction est de préférence conduite à une température comprise entre 50 et 120°C et de préférence selon un mode d'extraction à contre-courant. De la colonne d'extraction on extrait une coupe aromatique contenant le mélange de n-propanol et d'éthanol qui est ensuite envoyée dans une unité de distillation dont la finalité est de séparer le solvant d'extraction du mélange d'alcools.

D'autres caractéristiques et avantages de l'invention seront mieux compris et apparaîtront clairement à la lecture de la description faite ci-après en se référant aux dessins parmi lesquels :

- la figure 1 schématise un premier mode de réalisation du procédé selon l'invention;
- la figure 2 représente schématiquement un second mode de réalisation du procédé selon l'invention.

Généralement, les éléments semblables sont dénotés par des références identiques dans les figures.

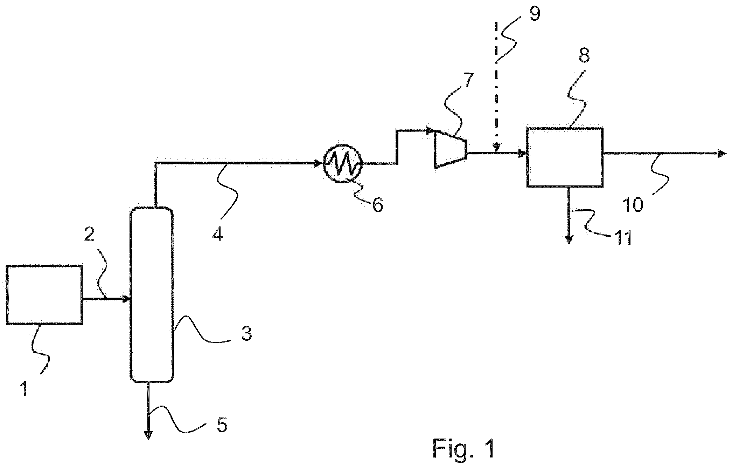

En référence à la figure 1, un effluent aqueux contenant un mélange d'éthanol, de n-propanol qui est issu d'une unité d'hydrogénation 1 d'une solution de propanoate d'éthyle, est envoyé par la ligne 2 vers une unité de séparation 3, qui est dans l'exemple de la figure 1 une colonne de distillation. L'objectif de cette première étape, que l'on peut qualifier de « séchage partiel », est de réduire la teneur en eau de l'effluent.

Le flux 2 entrant dans la colonne de distillation 3 peut ainsi comprendre jusqu'à 98% poids en eau.

En référence à la figure 1, on extrait de la colonne de distillation 3 une fraction aqueuse qui est évacuée en fond de la colonne par la ligne 5 et une fraction de tête renfermant un mélange aqueux d'alcools par la ligne 4. Le mélange aqueux d'alcool a typiquement une teneur massique en eau par rapport au poids total du mélange aqueux pouvant aller jusqu'à 50% poids. La colonne de distillation 3 est conçue et opérée de manière à récupérer de préférence 99% poids des alcools en tête de la colonne

Le mélange comprenant de l'eau, du n-propanol et l'éthanol est chauffée et vaporisée, grâce à un échangeur de chaleur 6 et ensuite comprimée grâce à un compresseur 7 avant d'être envoyé à l'étape de déshydratation, dans l'unité de déshydratation 8, afin de convertir lesdits alcools en éthylène et propylène.

La réaction de déshydratation est effectuée dans une unité de déshydratation 8 comprenant au moins un réacteur dans lequel on met en contact l'éthanol et le n-propanol avec un catalyseur capable de réaliser la déshydratation de l'éthanol et du n-propanol. Dans le cas où le mélange d'éthanol et de n-propanol a une teneur en eau inférieure à 30% poids par rapport au poids total du mélange, il est prévu d'apporter de l'eau audit mélange avant de le déshydrater. A cette fin il est prévu, comme indiqué sur la figure 1, une ligne 9 disposée en amont de l'unité de déshydratation 8 permettant d'injecter de l'eau au mélange en sortie du compresseur 7. De préférence, l'eau est apportée sous forme vapeur au mélange d'éthanol et de n-propanol.

Conformément à l'invention, cette étape de conversion de l'éthanol et du n-propanol en oléfines met en oeuvre une alumine choisie parmi:

- une alumine (A) ayant une surface spécifique BET mesurée selon la norme ASTM D 3663-03 comprise entre 200 et 350 $m^2$/g, un diamètre moyen mésoporeux compris entre 5 et 15 nm, une teneur en sodium inférieure à 50 ppm poids et une teneur en soufre inférieure à 40 ppm poids; et
- une alumine (B) une surface spécifique BET mesurée selon la norme ASTM D 3663-03 comprise entre 130 et 180 $m^2$/g, un diamètre moyen mésoporeux compris entre 14 et 20 nm, une teneur en sodium comprise entre 300 et 600 ppm poids et une teneur en soufre comprise entre 800 et 1300 ppm poids;

Il est à noter que la réaction de déshydratation en présence du catalyseur selon l'invention ne nécessite pas un séchage poussé du mélange d'éthanol/n-propanol dans la mesure où sa teneur en eau est comprise entre 30 à 75% poids par rapport au poids total du mélange. L'eau contenue dans le mélange d'alcools est avantageusement utilisée dans le procédé de déshydratation selon l'invention comme fluide thermique.

La réaction de déshydratation catalytique est réalisée à une température comprise entre 350 et 500°C, à une pression totale comprise entre 0,2 et 2 MPa et avec une vitesse pondérale horaire (pph) définie comme étant le rapport du débit massique en éthanol et n-propanol sur la masse de catalyseur comprise entre 1 et 10 h$^{-1}$.

L'étape de déshydratation est réalisée dans une unité qui comprend un ou plusieurs réacteurs disposés successivement. Le(s) réacteur(s) est (sont) pourvu(s) du catalyseur selon l'invention, qui est de préférence sous forme d'extrudés et fonctionne(nt) soit en lit fixe soit en lit mobile, mais préférentiellement en lit fixe.

L'étape de déshydratation des alcools est mise en oeuvre de préférence selon le procédé décrit dans la demande de brevet FR 2961202 qui met en jeu au moins un réacteur de déshydratation adiabatique. Le procédé implique que l'effluent issu du dernier réacteur de déshydratation est envoyé à l'échangeur de chaleur et que la charge à déshydrater est introduite dans l'étape préalable de vaporisation à une pression inférieure à la pression de l'effluent en sortie du dernier réacteur de déshydratation, de manière à maximiser l'échange de chaleur entre la charge et l'effluent issu du dernier réacteur, c'est à dire à échanger la totalité de l'enthalpie de vaporisation de la charge et l'enthalpie de condensation dudit effluent.

A l'issue de l'étape de déshydratation, on récupère en sortie de l'unité de déshydratation 8 et après une étape de distillation un effluent gazeux 10 contenant un mélange de propylène et d'éthylène et un effluent comprenant de l'eau par la ligne 11. Cet effluent gazeux de propylène et d'éthylène peut par la suite subir une étape de distillation en vue de séparer les deux oléfines, qui sont ensuite valorisable en comme matière première en pétrochimie, notamment dans l'industrie des polymères.

Comme mentionné ci-avant le procédé selon l'invention est avantageux du point de vue économique car il permet de produire de l'éthylène et du propylène à partir d'un mélange aqueux de n-propanol et d'éthanol en s'affranchissant d'une étape couteuse de séparation des alcools. Grâce au procédé selon l'invention, on convertit de façon concomitante l'éthanol et le n-propanol afin de produire un flux d'éthylène en mélange avec du propylène qui sont des oléfines facilement séparables notamment par distillation.

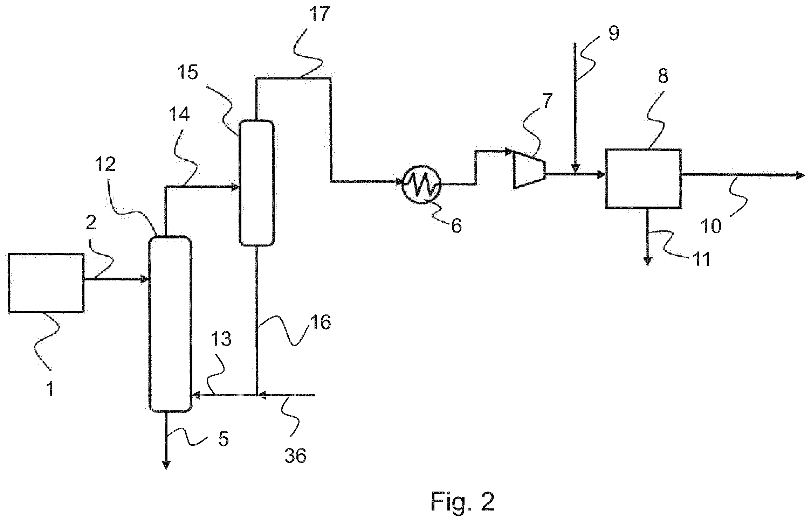

La figure 2 représente un second mode de réalisation du procédé selon l'invention qui diffère de celui de la figure 1 par le fait que l'unité de séparation de l'eau (séchage) est une unité d'extraction par solvant comprenant une colonne d'extraction liquide-liquide complétée de préférence par une colonne de distillation.

En référence à la figure 2, le jus fermentaire contenant de l'éthanol, du n-propanol et de l'eau , issu de l'unité 1 de fermentation est envoyé par la ligne 2 dans une colonne d'extraction liquide-liquide 12 et dans lequel on injecte, de préférence à contre-courant, par exemple au niveau du fond de la colonne d'extraction 12, via la ligne 13, un solvant qui est une coupe aromatique comprenant un mélange de composés aromatiques ayant 7 à 10 atomes de carbone. Le solvant selon l'invention a la capacité d'absorber l'éthanol et le n-propanol et présente en outre une solubilité quasiment nulle dans l'eau. Par exemple le solvant est un réformat lourd issu d'une unité de reformage catalytique des essences qui traite une coupe naphta de pétrole brut pour en augmenter la teneur en composés aromatiques. Cette étape de reformage catalytique (aromatisation de l'essence) s'accompagne d'un alourdissement de cette essence. Aussi, afin de garantir un point final d'ébullition de l'essence conforme à la spécification commerciale, il est usuel de redistiller ces essences pour éliminer la fraction la plus lourde qui est appelée « réformat lourd ».

Par différence de densité et grâce à l'affinité du solvant vis-à-vis de l'éthanol et du n-propanol, on récupère en tête de la colonne d'extraction un effluent organique 14 contenant la coupe aromatique et les alcools recherchés et en fond, via la ligne 5, un flux d'eau. Pour la mise en oeuvre de l'extraction liquide-liquide, le rapport massique entre la charge et le solvant est généralement compris entre 0,5 et 5. La mise en contact avec le solvant est réalisée généralement à une température comprise entre de

60 et 120°C et à une pression comprise entre 0,5 et5 MPa. Comme représenté à la figure 2, une ligne 15 permet de faire un appoint de solvant si cela est nécessaire pour le fonctionnement de la colonne 30. On récupère ainsi en tête de la colonne d'extraction liquide-liquide un effluent contenant au moins 99% poids des alcools présents dans le mélange d'alcools.

Conformément à la figure 2, l'effluent organique extrait en tête de la colonne d'extraction liquide-liquide 12 est envoyé dans une colonne de distillation 15 configurée pour séparer en tête le mélange comprenant l'éthanol et le n-propanol du solvant d'extraction. Ainsi le mélange d'alcools est récupéré par la ligne 17 tandis que la coupe aromatique est soutirée en fond de ladite colonne de distillation 15 par la ligne 16 et est recyclé via la ligne 13 dans la colonne d'extraction liquide-liquide 12. Cette distillation est de préférence réalisée à pression réduite, comprise de préférence entre 0,02 et 0,05 MPa afin de diminuer la température en fond de la colonne et ainsi pouvoir utiliser une vapeur "basse pression" comme fluide de rebouillage. Le procédé selon le mode de réalisation de la figure 2 présente l'avantage d'être moins énergivore par rapport au premier mode de réalisation car contrairement à une distillation, la mise en oeuvre de la colonne d'extraction liquide-liquide ne requiert pas de chauffer fortement le mélange d'alcools pour le sécher.

Dans le mode de réalisation de la figure 2, l'effluent soutiré par la ligne 17 qui est constitué essentiellement d'éthanol et de n-propanol est chauffé par l'échangeur 6 puis comprimé par le compresseur 7 avant d'être envoyé avec de l'eau apportée par la ligne 9 dans l'unité de déshydratation 13. L'appoint d'eau, via la ligne 9, permet de régler la teneur en eau de la charge d'alcools de manière à fournir un mélange éthanol-n-propanol-eau dont la teneur en eau est comprise entre 30 et 75% poids d'eau par rapport au poids total du mélange.

**Exemples**

Différents catalyseurs sont testés pour la déshydratation d'un mélange contenant 25% poids d'éthanol, 25% poids de n-propanol et 50% poids d'eau par rapport au poids total du mélange. Les catalyseurs ont la forme d'extrudés trilobes de 1,6 mm de diamètre.

Le tableau 1 ci-dessous donne les caractéristiques des catalyseurs testés.

Tableau 1

| Catalyseur | Type d'alumine | Diamètre moyen mésoporeux (nm) | Surface spécifique ($m^2$/g) mesurée selon ASTM D 3663-03 | Teneur en sodium (ppm poids) | Teneur en soufre (ppm poids) |
|---|---|---|---|---|---|
| C1 (selon l'invention) | Alumine $\gamma$ pseudo-cubique | 10,3 | 208 | Inférieure à 20 | Inférieure à 40 |
| C2 (selon l'invention) | Alumine $\gamma$ cubique | 8,5 | 216 | 28 | Inférieure à 40 |
| C3 (selon l'invention) | Alumine $\gamma$ cubique | 7,4 | 232 | 35 | Inférieure à 40 |
| C4 (comparatif) | Alumine $\gamma$ cubique et alumine $\chi$ | 5,7 | 155 | 417 | Inférieure à 40 |
| C5 (comparatif) | Alumine $\gamma$ cubique | 8,7 | 270 | 394 | 1262 |
| C6 (selon l'invention) | Alumine $\gamma$ cubique | 15,7 | 175 | 550 | 903 |
| C7 (selon l'invention) | Alumine $\gamma$ tétragonal + alumine $\theta$ | 18,3 | 152 | 448 | 1210 |
| C8 (comparatif) | alumine $\delta$ et alumine $\chi$ | 25,8 | 154 | 401 | Inférieure à 40 |
| C9 (comparatif) | Alumine $\gamma$ cubique | 18,3 | 143 | 222 | 1121 |

Les catalyseurs C1, C2 et C3 sont conformes à une alumine (A) selon l'invention et les catalyseurs C6 et C7 sont conformes à une alumine (B) selon l'invention.

Le catalyseur C4 est non-conforme à une alumine (A) selon l'invention car sa surface spécifique et sa teneur en sodium sont en dehors des gammes revendiquées. En outre le catalyseur D est non-conforme à une alumine (B) selon l'invention car son diamètre moyen mésoporeux est en dehors de la gamme 14-20 nm.

Le catalyseur C5 non-conforme se distingue d'une alumine (A) selon l'invention par les teneurs en sodium et soufre qui sont plus hautes que la limite supérieure des gammes revendiquées. Le catalyseur C5 est également non-conforme à une alumine (B) selon l'invention et s'en distingue par son diamètre moyen mésoporeux et sa surface spécifique.

Le catalyseur C8 ne répond pas à la définition d'une alumine (A) selon l'invention car le diamètre moyen mésoporeux, la surface spécifique et la teneur en sodium sont en dehors des gammes revendiquées. Le catalyseur C8 est par ailleurs non-conforme à une alumine (B) selon l'invention car il présente un diamètre moyen mésoporeux supérieur à la gamme revendiquée et une teneur en soufre inférieur à 800 ppm poids

Le catalyseur C9 est non-conforme à une alumine (A) selon l'invention par son diamètre moyen mésoporeux, sa surface spécifique, sa teneur en sodium et en soufre. Le catalyseur I se distingue d'une alumine (B) selon l'invention par sa teneur en sodium.

Avant leur mise en oeuvre dans le test de déshydratation du mélange d'alcools, tous les catalyseurs ont subi une phase de conditionnement qui comprend les étapes suivantes :

i) chauffage du catalyseur d'une température ambiante jusqu'à 550°C sous flux d'air à 5 Nl/h avec une rampe de température de 20°C par heure ;
ii) maintien à une température de 550°C sous débitd'air pendant 2 heures ;
iii) refroidissement du catalyseur jusqu'à la température du test sous un flux d'azote à 100 Nl/h ;
iv) maintien du catalyseur à la température de test sous un flux d'azote à 100 Nl/h pendant 5 heures.

La réaction de déshydratation est réalisée dans un réacteur isotherme contenant un lit fixe de catalyseur de déshydratation. Tous les catalyseurs ont été testés dans les mêmes conditions opératoires à savoir :

- température dans le réacteur = 400°C ;
- pression relative dans le réacteur = 0,2 MPa ;
- vitesse pondérale horaire (pph), qui est définie comme étant le rapport du débit massique en (éthanol + n-propanol) sur la masse de catalyseur = 2 h$^{-1}$.

L'effluent en sortie du réacteur de déshydratation est envoyé dans un séparateur haute pression (HP) pour séparer un premier flux gazeux et un flux liquide. Le flux liquide est ensuite transféré dans un séparateur basse pression (BP) d'où l'on sépare un second flux gazeux et un second flux liquide. Les deux flux gazeux sont mélangés avant d'être analysés.
Les flux liquide et gazeux sont analysés par chromatographie en phase gaz.
Le tableau 2 donne les performances catalytiques des alumines testées après 72 heures de test.
Les conversions et les sélectivités ont été calculées à partir des formules décrites ci-avant.
Enfin, la pureté de la coupe C2 est calculée par rapport à l'éthylène, l'éthane et le méthane tandis que la pureté de la coupe C3 est calculée par rapport au propylène, au propane et au cyclopropane. Ainsi les puretés en éthylène de la coupe C2 et en propylène de la coupe C3 s'expriment de la manière suivante :

$$\text{Pureté éthylène} = \left( \frac{mC_{\text{éthylène sortie}}}{mC_{\text{éthylène sortie}} + mC_{\text{éthane sortie}} + mC_{\text{méthane sortie}}} \right) * 100$$

$$\text{Pureté propylène} = \left( \frac{mC_{\text{propylène sortie}}}{mC_{\text{propylène sortie}} + mC_{\text{propane sortie}} + mC_{\text{cyclopropane sortie}}} \right) * 100$$

Tableau 2

| Catalyseur | C1 | C2 | C3 | C4 | C5 | C6 | C7 | C8 | C9 |
|---|---|---|---|---|---|---|---|---|---|
| **Conversion du n-Propanol (%)** | **99,98** | **99,99** | **99,99** | 98,80 | 99,50 | **99,99** | **99,97** | 95,76 | 99,55 |
| Sélectivité en Propylène | **99,96** | **99,74** | **99,56** | 98,84 | 99,02 | **99,29** | **99,44** | 97,86 | 97,75 |
| Sélectivité en Dipropylether | 0 | 0 | 0 | 0,05 | 0 | 0 | 0 | 0,14 | 0,02 |
| Sélectivité en Propane | 0,06 | 0,06 | 0,12 | 0,18 | 0,22 | 0,11 | 0,08 | 0,06 | 1,02 |
| Sélectivité en Propanal | 0,13 | 0,17 | 0,16 | 0,16 | 0,17 | 0,25 | 0,18 | 0,05 | 0,55 |
| Sélectivité en Ethoxypropane | 0 | 0 | 0 | 0,12 | 0,09 | 0 | 0 | 1,35 | 0,07 |
| Sélectivité en Cyclopropane | 0,14 | 0,04 | 0,02 | 0,65 | 0,50 | 0,35 | 0,30 | 0,54 | 0,59 |
| **Composition coupe C3 (% poids)** | | | | | | | | | |
| Propylène | **99,79** | **99,91** | **99,86** | 99,17 | 99,28 | **99,54** | **99,62** | 99,39 | 98,38 |
| Propane | 0,07 | 0,03 | 0,09 | 0,2 | 0,21 | 0,12 | 0,07 | 0,07 | 0,97 |
| Cyclopropane | 0,15 | 0,04 | 0,02 | 0,65 | 0,50 | 0,35 | 0,30 | 0,55 | 0,59 |
| **Conversion de l'éthanol (%)** | **99,34** | **99,88** | **99,56** | 91,99 | 95,47 | **99,70** | **99,56** | 65,20 | 98,20 |
| Sélectivité méthane | 0,00 | 0,00 | 0,00 | 0,00 | 0,00 | 0,00 | 0,00 | 0,00 | 0,00 |
| Sélectivité Ethylène | **99,57** | **99,66** | **99,49** | 98,14 | 98,68 | **99,53** | **99,58** | 84,55 | 96,79 |

(suite)

| Catalyseur | C1 | C2 | C3 | C4 | C5 | C6 | C7 | C8 | C9 |
|---|---|---|---|---|---|---|---|---|---|
| Sélectivité Ethane | 0,16 | 0,13 | 0,19 | 0,25 | 0,26 | 0,18 | 0,15 | 0,25 | 1,20 |
| Sélectivité Diéthylether | 0 | 0 | 0 | 1,00 | 0,55 | 0 | 0 | 13,17 | 0,40 |
| Sélectivité EthylPropylEther | 0 | 0 | 0 | 0,20 | 0,11 | 0 | 0 | 1,82 | 0,08 |
| Sélectivité Butane | 0,13 | 0,10 | 0,14 | 0,17 | 0,16 | 0,10 | 0,09 | 0,16 | 0,50 |
| Sélectivité Butène | 0,11 | 0,09 | 0,15 | 0,21 | 0,15 | 0,13 | 0,13 | 0,03 | 0,68 |
| Sélectivité C5+ | 0,02 | 0,02 | 0,02 | 0,03 | 0,09 | 0,05 | 0,04 | 0,02 | 0,35 |
| **Composition coupe C2 (% poids)** | | | | | | | | | |
| Ethylène | **99,84** | **99,87** | **99,81** | 99,75 | 99,74 | **99,82** | **99,85** | 99,71 | 98,78 |
| Ethane | 0,16 | 0,13 | 0,19 | 0,25 | 0,26 | 0,18 | 0,15 | 0,29 | 1,22 |
| Méthane | 0,00 | 0,00 | 0,00 | 0,00 | 0,00 | 0,00 | 0,00 | 0,00 | 0,00 |

La mise en oeuvre des catalyseurs selon l'invention (C1, C2, C3, C6 et C7) permettent d'obtenir d'excellents taux de conversion du n-propanol mais également de l'éthanol qui sont supérieurs à 99%. En revanche on constante qu'avec les catalyseurs hors invention (D, E, H, I) les taux de conversion de l'éthanol sont inférieurs à 99%.
Par ailleurs on observe que la pureté du propylène dans une coupe C3 et celle de l'éthylène dans une coupe C2 sont meilleures lorsque les catalyseurs selon l'invention sont mis en oeuvre. Les coupes C2 et C3 ainsi produites sont de grade polymère et peuvent donc être utilisées dans l'industrie pétrochimique notamment pour la fabrication de polymères.

**Revendications**

1. Procédé de production d'un mélange d'éthylène et de propylène à partir d'un mélange contenant de l'éthanol, du n-propanol et ayant une teneur en eau comprise entre 30 et 75% poids par rapport au poids total du mélange, dans lequel:

   a) on met en contact dans une unité de déshydratation le mélange avec un catalyseur de déshydratation choisi parmi:

   - une alumine (A) ayant une surface spécifique BET mesurée selon la norme ASTM D 3663-03 comprise entre 200 et 280 m$^2$/g, un diamètre moyen mésoporeux compris entre 5 et 15 nm, une teneur en sodium inférieure à 50 ppm poids et une teneur en soufre inférieure à 40 ppm poids; et
   - une alumine (B) ayant une surface spécifique BET mesurée selon la norme ASTM D 3663-03 comprise entre 130 et 180 m$^2$/g, un diamètre moyen mésoporeux compris entre 14 et 20 nm, une teneur en sodium comprise entre 300 et 600 ppm poids et une teneur en soufre comprise entre 800 et 1300 ppm poids;

   la mise en contact étant effectuée à une température comprise entre 350 et 500°C, à une pression totale comprise entre 0,2 et 2 MPa et avec une vitesse pondérale horaire (pph) définie comme étant le rapport du débit massique en éthanol et n-propanol sur la masse de catalyseur comprise entre 1 et 10 h$^{-1}$,
   b) on soutire un effluent contenant de l'éthylène et du propylène de ladite unité de déshydratation.

2. Procédé selon la revendication 1, dans lequel le mélange contient entre 1 et 75% poids d'éthanol et entre 99 et 25% poids de n-propanol par rapport au poids total éthanol et n-propanol.

3. Procédé selon l'une des revendications 1 ou 2, dans lequel les alumines (A) et (B) sont des alumines gamma.

4. Procédé selon l'une des revendications précédentes, dans lequel l'alumine (A) a un diamètre moyen mésoporeux compris entre 6 et 12 nm et de préférence compris entre 7 et 11 nm.

5. Procédé selon l'une des revendications 1 à 3, dans lequel l'alumine (B) a une surface spécifique BET mesurée selon la norme ASTM D 3663-03 comprise entre 150 et 180 m$^2$/g.

**6.** Procédé selon l'une des revendications 1 à 3 ou selon la revendication 5, dans lequel l'alumine (B) a un diamètre moyen mésoporeux compris entre 15 et 20 nm.

**7.** Procédé selon l'une des revendications précédentes, dans lequel avant l'étape a) on met en contact dans une unité de séparation comprenant une colonne d'extraction liquide-liquide (12), le mélange avec une coupe aromatique comprenant un mélange de composés aromatiques ayant 7 à 10 atomes de carbone de manière à séparer de ladite colonne d'extraction (12) une fraction aqueuse (5) et une fraction organique (14) contenant la coupe aromatique, de l'éthanol, du n-propanol et on envoie ladite fraction organique (14) dans une colonne de distillation (15) configurée pour fournir un effluent (16) contenant la coupe aromatique et un mélange (17) contenant de l'éthanol, du n-propanol et on envoie ledit mélange dans l'unité de déshydratation.

**8.** Procédé selon la revendication 7, dans lequel la coupe aromatique est un mélange de 1,3,5-triméthylbenzène et le 1,2,4-triméthylbenzène.

**9.** Procédé selon l'une des revendications 7 ou 8, dans lequel la mise en contact du mélange contenant de l'éthanol, du n-propanol, de l'eau avec la coupe aromatique est faite à contre-courant.

**10.** Procédé selon l'une des revendications 1 à 6, dans lequel avant l'étape a) on envoie le mélange contenant de l'éthanol, du n-propanol, de l'eau dans une unité de séparation comprenant une colonne de distillation (3) de manière à séparer de la colonne de distillation une fraction aqueuse et un effluent contenant un mélange d'éthanol, de n-propanol et de l'eau avec une teneur en eau comprise entre 30 et 75% poids par rapport au poids total du mélange et on envoie l'effluent dans l'unité de déshydratation.

**11.** Procédé selon l'une des revendications précédentes dans lequel le mélange contenant de l'éthanol et du n-propanol est issue d'une hydrogénation du propanoate d'éthyle.

**12.** Procédé selon la revendication 11, dans lequel le propanoate d'éthyle est obtenue par estérification de l'acide propanoïque avec l'éthanol, l'acide propanoïque et l'éthanol étant d'origine biologique.

**13.** Procédé selon l'une des revendications précédentes, dans lequel la mise en contact de l'étape a) est réalisée dans au moins un réacteur à une température comprise entre 350 et 450°C et de manière plus préférée comprise entre 375 et 425°C, à une pression totale comprise entre 0,2 et 1 MPa et de manière plus préférée comprise entre 0,2 et 0,7 MPa, et avec une vitesse pondérale horaire (pph) comprise entre 2 et 8 h$^{-1}$.

**14.** Procédé selon l'une des revendications précédentes dans lequel l'étape a) est effectuée dans deux réacteurs adiabatiques en série.

**Patentansprüche**

**1.** Verfahren zur Herstellung einer Mischung von Ethylen und Propylen aus einer Mischung, die Ethanol, n-Propanol enthält und einen Wassergehalt zwischen 30 und 75 Gew.-%, bezogen auf das Gesamtgewicht der Mischung, aufweist, wobei:

a) in einer Dehydratationseinheit die Mischung mit einem Dehydratationskatalysator in Berührung gebracht wird, ausgewählt aus:

- einem Aluminiumoxid (A) mit einer spezifischen Oberfläche BET, gemessen gemäß der Norm ASTM D 3663-03, zwischen 200 und 280 m$^2$/g, einem mesoporösen mittleren Durchmesser zwischen 5 und 15 nm, einem Natriumgehalt von weniger als 50 Gew.-ppm, und einem Schwefelgehalt von weniger als 40 Gew.-ppm; und
- einem Aluminiumoxid (B) mit einer spezifischen Oberfläche BET, gemessen gemäß der Norm ASTM D 3663-03, zwischen 130 und 180 m$^2$/g, einem mesoporösen mittleren Durchmesser zwischen 14 und 20 nm, einem Natriumgehalt zwischen 300 und 600 Gew.-ppm, und einem Schwefelgehalt zwischen 800 und 1300 Gew.-ppm;

wobei das Inberührungbringen bei einer Temperatur zwischen 350 und 500 °C, bei einem Gesamtdruck zwischen 0,2 und 2 MPa und mit einer stündlichen Gewichtsgeschwindigkeit (pph), die als Verhältnis des Massendurchsatzes von Ethanol und n-Propanol zur Masse des Katalysators definiert wird, zwischen 1 und 10 h$^{-1}$ erfolgt,
b) ein Abfluss, der Ethylen und Propylen enthält, aus der Dehydratationseinheit abgezogen wird.

2. Verfahren nach Anspruch 1, wobei die Mischung zwischen 1 und 75 Gew.-% Ethanol und zwischen 99 und 25 Gew.-% n-Propanol, bezogen auf das Gesamtgewicht von Ethanol und n-Propanol, enthält.

3. Verfahren nach Anspruch 1 oder 2, wobei die Aluminiumoxide (A) und (B) gamma-Aluminiumoxide sind.

4. Verfahren nach einem der vorhergehenden Ansprüche, wobei das Aluminiumoxid (A) einen mesoporösen mittleren Durchmesser zwischen 6 und 12 nm und vorzugsweise zwischen 7 und 11 nm aufweist.

5. Verfahren nach einem der Ansprüche 1 bis 3, wobei das Aluminiumoxid (B) eine spezifische Oberfläche BET, gemessen gemäß der Norm ASTM D 3363-03, zwischen 150 und 180 m$^2$/g aufweist.

6. Verfahren nach einem der Ansprüche 1 bis 3 oder nach Anspruch 5, wobei das Aluminiumoxid (B) einen mesoporösen mittleren Durchmesser zwischen 15 und 20 nm aufweist.

7. Verfahren nach einem der vorhergehenden Ansprüche, wobei vor Schritt a) in einer Trenneinheit, die eine Flüssig-Flüssig-Extraktionssäule (12) umfasst, die Mischung mit einem aromatischen Schnitt, der eine Mischung aromatischer Verbindungen mit 7 bis 10 Kohlenstoffatomen umfasst, in Berührung gebracht wird, um von der Extraktionssäule (12) eine wässrige Fraktion (5) und eine organische Fraktion (14) zu trennen, die den aromatischen Schnitt, Ethanol, n-Propanol enthält, und die organische Fraktion (14) in eine Destillationssäule (15) geschickt wird, die ausgelegt ist, einen Abfluss (16) zu liefern, der den aromatischen Schnitt und eine Mischung (17) enthält, die Ethanol, n-Propanol enthält, und die Mischung in eine Dehydratationseinheit geschickt wird.

8. Verfahren nach Anspruch 7, wobei der aromatische Schnitt eine Mischung von 1,3,5-Trimethylbenzol und 1,2,4-Trimethylbenzol ist.

9. Verfahren nach einem der Ansprüche 7 oder 8, wobei das Inberührungbringen der Mischung, die Ethanol, n-Propanol, Wasser enthält, mit dem aromatischen Schnitt im Gegenstrom erfolgt.

10. Verfahren nach einem der Ansprüche 1 bis 6, wobei vor dem Schritt a) die Mischung, die Ethanol, n-Propanol, Wasser enthält, in eine Trenneinheit geschickt wird, die eine Destillationssäule (3) umfasst, um von der Destillationssäule eine wässrige Fraktion und einen Abfluss zu trennen, der eine Mischung von Ethanol, n-Propanol und Wasser mit einem Wassergehalt zwischen 30 und 75 Gew.-%, bezogen auf das Gesamtgewicht der Mischung, enthält, und der Abfluss in eine Dehydratationseinheit geschickt wird.

11. Verfahren nach einem der vorhergehenden Ansprüche, wobei die Mischung, die Ethanol und n-Propanol enthält, aus einer Hydrierung von Ethylpropanoat stammt.

12. Verfahren nach Anspruch 11, wobei das Ethylpropanoat durch Veresterung von Propansäure mit Ethanol erhalten wird, wobei die Propansäure und das Ethanol biologischen Ursprungs sind.

13. Verfahren nach einem der vorhergehenden Ansprüche, wobei das Inberührungbringen in Schritt a) in mindestens einem Reaktor bei einer Temperatur zwischen 350 und 450 °C und bevorzugter zwischen 375 und 425 °C, bei einem Gesamtdruck zwischen 0,2 und 1 MPa und bevorzugter zwischen 0,2 und 0,7 MPa, und mit einer stündlichen Gewichtsgeschwindigkeit (pph) zwischen 2 und 8 h$^{-1}$ erfolgt.

14. Verfahren nach einem der vorhergehenden Ansprüche, wobei Schritt a) in zwei adiabatischen Reaktoren in Serie durchgeführt wird.

## Claims

1. Process for the production of a mixture of ethylene and propylene from a mixture containing ethanol and n-propanol and having a water content comprised between 30 and 75% by weight with respect to the total weight of the mixture, in which:

a) the mixture is brought into contact, in a dehydration unit, with a dehydration catalyst chosen from:

- an alumina (A) having a BET specific surface area measured according to the standard ASTM D 3663-03 comprised between 200 and 280 m²/g, a mean mesopore diameter comprised between 5 and 15 nm, a sodium content of less than 50 ppm by weight and a sulphur content of less than 40 ppm by weight; and
- an alumina (B) having a BET specific surface area measured according to the standard ASTM D 3663-03 comprised between 130 and 180 m²/g, a mean mesopore diameter comprised between 14 and 20 nm, a sodium content comprised between 300 and 600 ppm by weight and a sulphur content comprised between 800 and 1300 ppm by weight;

the bringing into contact being carried out at a temperature comprised between 350 and 500°C, at a total pressure comprised between 0.2 and 2 MPa and with a weight hourly space velocity (whsv) defined as being the ratio of the mass flow rate of ethanol and n-propanol to the mass of catalyst comprised between 1 and 10 h$^{-1}$,
b) an effluent containing ethylene and propylene is drawn off from said dehydration unit.

2. Process according to claim 1, in which the mixture contains between 1 and 75% by weight of ethanol and between 99 and 25% by weight of n-propanol with respect to the total weight of ethanol and n-propanol.

3. Process according to one of claims 1 or 2, in which the aluminas (A) and (B) are gamma aluminas.

4. Process according to one of the preceding claims, in which the alumina (A) has a mean mesopore diameter comprised between 6 and 12 nm and preferably comprised between 7 and 11 nm.

5. Process according to one of claims 1 to 3, in which the alumina (B) has a BET specific surface area measured according to the standard ASTM D 3663-03 comprised between 150 and 180 m²/g.

6. Process according to one of claims 1 to 3 or according to claim 5, in which the alumina (B) has a mean mesopore diameter comprised between 15 and 20 nm.

7. Process according to one of the preceding claims, in which before stage a) the mixture is brought into contact with an aromatic cut comprising a mixture of aromatic compounds having 7 to 10 carbon atoms, in a separation unit comprising a liquid-liquid extraction column (12), so as to separate from said extraction column (12) an aqueous fraction (5) and an organic fraction (14) containing the aromatic cut, ethanol, n-propanol and said organic fraction (14) is sent into a distillation column (15) configured in order to provide an effluent (16) containing the aromatic cut and a mixture (17) containing ethanol and n-propanol and said mixture is sent into the dehydration unit.

8. Process according to claim 7, in which the aromatic cut is a mixture of 1,3,5-trimethylbenzene and 1,2,4-trimethylbenzene.

9. Process according to one of claims 7 or 8, in which the mixture containing ethanol, n-propanol and water is brought into contact with the aromatic cut is carried out in counter-current.

10. Process according to one of claims 1 to 6, in which before stage a) the mixture containing ethanol, n-propanol and water is sent into a separation unit comprising a distillation column (3) so as to separate from the distillation column an aqueous fraction and an effluent containing a mixture of ethanol, n-propanol and water with a water content comprised between 30 and 75% by weight with respect to the total weight of the mixture and the effluent is sent into the dehydration unit.

11. Process according to one of the preceding claims in which the mixture containing ethanol and n-propanol originates from a hydrogenation of ethyl propanoate.

12. Process according to claim 11, in which the ethyl propanoate is obtained by esterification of propanoic acid with ethanol, the propanoic acid and the ethanol being of biological origin.

13. Process according to one of the preceding claims, in which the bringing into contact of stage a) is carried out in at least one reactor at a temperature comprised between 350 and 450°C and more preferably comprised between 375 and 425°C, at a total pressure comprised between 0.2 and 1 MPa and more preferably comprised between 0.2 and 0.7 MPa, and with a weight hourly space velocity (whsv) comprised between 2 and 8 h$^{-1}$.

**14.** Process according to one of the preceding claims in which stage a) is carried out in two adiabatic reactors in series.

Fig. 1

Fig. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004078336 A
- WO 2011162717 A
- FR 2961202
- FR 2978146

**Littérature non-brevet citée dans la description**

- **H. KNÖZINGER ; R. KÖHNE.** The Deshydration of Alcohols over Alumina. I: The reaction scheme. *Journal of Catalysis,* 1966, vol. 5, 264-270
- **S.N. CHAUDHURI.** Reactions of ethanol over ZSM-5. *Journal of Molecular Catalysis,* 1990, vol. 62, 289-295
- **P. EUZEN ; P. RAYBAUD ; X. KROKIDIS ; H. TOULHOAT ; J.L. LE LOARER ; J.P. JOLIVET ; C. FROIDEFOND ; ALUMINA.** Handbook of Porous Solids. Wiley-VCH, 2002, 1591-1677